# EUROPÄISCHE PATENTSCHRIFT

(11) **EP 2 157 984 B1**
(45) Veröffentlichungstag und Bekanntmachung des Hinweises auf die Patenterteilung: **26.02.2014**
(21) Anmeldenummer: 08773442.2
(22) Anmeldetag: 14.06.2008
(51) Int. Cl.: A61M 1/16, A61M 1/34, A61N 1/18

(54) **MEDIZINISCHE BLUTBEHANDLUNGSEINRICHTUNG FÜR EINE EXTRAKORPORALE BLUTBEHANDLUNG MIT ELEKTRISCHER MUSKELSTIMULATION**
MEDICAL BLOOD TREATMENT DEVICE FOR AN EXTRACORPOREAL BLOOD TREATMENT WITH ELECTRIC MUSCLE STIMULATION
DISPOSITIF MÉDICAL DE TRAITEMENT DU SANG POUR UN TRAITEMENT EXTRACORPOREL DU SANG AVEC STIMULATION MUSCULAIRE ÉLECTRIQUE

(30) Priorität: 19.06.2007 DE 102007028133
(43) Veröffentlichungstag der Anmeldung: 03.03.2010
(73) Patentinhaber: Fresenius Medical Care Deutschland GmbH, 61352 Bad Homburg (DE)
(72) Erfinder: SCHRÖRS, Alexander, 60389 Frankfurt (DE)
(74) Vertreter: Oppermann, Frank
(86) Internationale Anmeldenummer: PCT/EP2008/004805
(87) Internationale Veröffentlichungsnummer: WO 2008/155077

(56) Entgegenhaltungen:
- EP-A- 1 205 144
- WO-A-02/13691
- US-A- 4 008 712
- US-A- 4 790 319
- US-A- 4 942 880

## Beschreibung

Die Erfindung bezieht sich auf eine medizinische Blutbehandlungseinrichtung für eine extrakorporale Blutbehandlung mit einem extrakorporalen Blutkreislauf. Darüber hinaus betrifft die Erfindung eine Vorrichtung zur extrakorporalen Blutbehandlung zur Verwendung mit einer Vorrichtung zur elektrischen Muskelstimulation sowie eine Vorrichtung zur elektrischen Muskelstimulation zur Verwendung mit einer extrakorporalen Blutbehandlungsvorrichtung.

Zur Entfernung von hampflichtigen Substanzen und zum Flüssigkeitsentzug werden bei chronischem Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von einer semipermeablen Membran getrennt sind. Während der Behandlung strömt das Blut des Patienten durch die Blutkammer, wobei zur Reinigung des Bluts von harnpflichtigen Substanzen durch die Dialysierflüssigkeitskammer des Dialysators Dialysierflüssigkeit strömt.

Während bei der Hämodialyse (HD) der Transport der kleineren molekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei einer extrakorporalen Blutbehandlung, beispielsweise einer Hämodialyse, stellen sich die folgenden Probleme. Während der Dialyse wird im Wesentlichen nur das Blut des Patienten gereinigt, nicht aber die zellulären und intrazellulären Kompartimente. Da der Transport der wassergelösten Toxine in das Blut nur sehr langsam erfolgt, werden mit einer Dialysebehandlung über eine längere Zeitdauer besser Ergebnisse als mit einer kurzen Dialyse erzielt. Aufgrund der effektiven Reinigung des Blutes und der schlechten Reinigung der zellulären und intrazellulären Kompartimente ergeben sich große Konzentrationsgradienten. Unmittelbar nach der Dialyse kommt es daher zu einem Konzentrationsausgleich der Toxine zwischen den Kompartimente und dem Blut, was auch als Rebound bezeichnet wird. Der Rebound stellt eine hohe Belastung für den Kreislauf des Patienten dar. In einzelnen Fällen kann der Rebound sogar zum Kreislaufversagen nach der Dialyse führen. Ein weiteres Problem ergibt sich daraus, dass die Dialysebehandlung im Sitzen oder Liegen durchgeführt werden muss, weil der Aktionsradius der Patienten durch die Dialysemaschine begrenzt wird. Körperliche Ruhe führt aber zu einer behinderten Durchblutung der peripheren Teile des Körpers, insbesondere der Beine, weshalb das Blut dort schlechter gereinigt wird.

Auf dem Fachgebiet der Dialyse ist allgemein bekannt, dass sich regelmäßiger Sport des Patienten positiv auf die Dialysebehandlung auswirkt (Nephron 42: 311-316 (1986) Exercise Training Reduces Coronary Risk and Effektively Rehabilitates Hemodialysis Patients, Andrew P. Goldberg et al.).

Des Weiteren ist bekannt, dass die Effizienz einer Dialysebehandlung dadurch signifikant gesteigert werden kann, dass der Patient sich während der Dialysebehandlung körperlich aktiv betätigt. Dadurch lassen sich auch die sog. Rebound-Effekte verringern. Es ist bekannt, dass eine körperliche Betätigung des Patienten während der Dialysebehandlung zu einer verbesserten Durchblutung des Körpers führt, so dass die nur schwach durchbluteten Bereiche des Körpers besser durchblutet werden, wodurch die Reinigung des Bluts verbessert wird. Bei körperlicher Betätigung während der Dialyse haben sich neben einem leichten Anstieg der Herzrate und des Blutdrucks ein verbesserter Stoffaustausch zwischen den Kompartimenten sowie ein Anstieg der Clearance gezeigt, was gleichbedeutend mit einer längeren Dialysebehandlung ist. Auch zeigt sich eine Reduzierung des Rebounds von Harnstoff, Kreatinin und Kalium (Nephron 43: 87-92 (1986), Effects of Exercise Training during Hämodialyses, Patricia L. Painter et al.; Nephrol Dial Transplant (1999) 14: 2927-2931, The effect of exercise during haemodialysis on solute removal, Chiew H. Kong et al.)

Als den Kreislauf anregende und die Durchblutung fördernde Maßnahmen sind auch Massagen insbesondere der Beine des Patienten bekannt, die sich aber als personal- und kostenintensiv erweisen. Im Übrigen sind die Ergebnisse mit Massagen nur schwer reproduzierbar.

Die Untersuchungen zum Einfluss sportlicher Betätigung auf die Ergebnisse von Dialysebehandlungen sind bei Patienten durchgerührt worden, die eine ausreichende körperliche Leistungsfähigkeit besaßen. Aufgrund des hohen Durchschnittsalters der Dialysepatienten, das bei ca. 67 Jahren liegt, verfügen viele Dialysepatienten aber nicht mehr über eine ausreichende körperliche Fitness, um selber aktiv sportliche Übungen während der Dialysebehandlung durchzuführen. Außerdem ist zu beachten, dass es bei Bewegungen des Patienten zu einer unfallmäßigen Diskonnektion der venösen und/oder der arteriellen Kanüle während der Dialyse kommen kann. Daher bedarf eine sportliche Betätigung des Dialysepatienten während der Dialysebehandlung einer verstärkten Überwachung, wobei höhere Anforderungen an die technischen Sicherheitsvorkehrungen zur schnellen Erkennung einer Nadeldiskonnektion gestellt werden müssen, was ebenfalls mit höheren Kosten verbunden ist.

Auf dem Gebiet der Sportmedizin ist es bekannt, durch eine so genannte elektrische Muskelstimulation, die auch als EMS bezeichnet wird, die Leistungsfähigkeit von Sportlern zu erhöhen. Dabei wird die gezielte elektrische Stimulation der Muskeln nicht nur zum Trainieren, sondern auch zum Aufwärmen, Entspannen und beschleunigten Regenerieren eingesetzt. Es ist auch bekannt, die elektrische Muskelstimulation (EMS) für therapeutische Maßnahmen einzusetzen.

Die US 2005/0131489 A1 beschreibt beispielsweise den Einsatz der elektrischen Muskelstimulation zur Vermeidung von venösen Thrombosen an unbeweglichen Patienten, beispielsweise in Intensivstationen. Eine Vorrichtung zur Muskelstimulation für den gleichen Anwendungsfall ist auch aus der WO 03/063960 A2 bekannt.

Der Erfindung liegt die Aufgabe zugrunde, eine medizinische Blutbehandlungseinrichtung bereit zu stellen, die eine extrakorporale Blutbehandlung mit höherer Effizienz erlaubt. Eine weitere Aufgabe der Erfindung ist, eine Vorrichtung zur extrakorporalen Blutbehandlung zur Verwendung mit einer Vorrichtung zur elektrischen Muskelstimulation sowie eine Vorrichtung zur elektrischen Muskelstimulation zur Verwendung mit einer extrakorporalen Blutbehandlungsvorrichtung bereit zu stellen, mit denen sich die Effizienz der extrakorporalen Blutbehandlung erhöhen lassen.

Die WO 02/13691 A beschreibt eine Vorrichtung und ein Verfahren zur bioelektrischen Impedanzanalyse BIA). Die bioelektrische Impedanzanalyse BIA) dient der Bestimmune des Flüssigkeitshaushalts mittels Wechselstroms. Die BIA wird auch zur Bestimmung des Körperfettanteils eingesetzt. Die bekannte Vorrichtung zur BIA verfügt über einen Mikroprozessor, ein Display sowie eine Messeinheit. An der Messeinheit sind Elektroden angeschlossen, die auf die Haut des Patienten aufgelegt werden. Die WO 02/13691 schlägt vor, die bioelektrische Impedanzanalyse während einer Dialysebehandlung durchzuführen. Zum Austausch von Daten verfügt die Vorrichtung zur BIA über eine Schnittstelle.

Die US-A-4 942 880 beschreibt ein Verfahren zur Diagnose von Veränderungen des Gewebes mittels elektrischer Impulse. Die US-A-4 942 880 schlägt als vorteilhaft vor, während der Dialysebehandlung bestimmte Bereiche des Körpers des Patienten zu behandeln, an denen Veränderungen am Gewebe aufgetreten sind. Aber auch nach der Dialysebehandlung soll das Gewebe noch behandelt werden, wo Symptome auftreten. Folglich befasst sich die US-A-4 942 880 mit der Diagnose und Behandlung von Begleiterscheinungen, die bei der Dialyse auftreten können (Nebenwirkungen).

Die US-A- 4 790 313 beschreibt eine Vorrichtung, die den pleichen Zweck wie die Vorrichtung der US-A-4 942 880 hat. Die bekannte Vorrichtung soll vor der eigentlichen Dialysebehandlung, aber möglicherweise auch während der Behandlung eingesetzt werden. Dabei soll sie wieder der Behandlung des Gewebes dienen.

Die US-A-4 008 712 beschreibt eine Vorrichtung, die zur Messunp, von elektrischen Eigenschaften an bestimmten Körperstellen dient, insbesondere soll eine Veränderung des TBW (Total Body Water) bestimmt werden. Auch aus der EP 1 205 144 A1 ist nur eine Vorrichtung bekannt, mit der das Trockengewicht bestimmt werden soll.

Die Lösung dieser Aufgaben erfolgt erfindungsgemäß mit den Merkmalen der Patentansprüche 1, 11 und 12. Vorteilhafte Ausführungsformen der Erfindung sind Gegenstand der Unteransprüche.

Die erfindungsgemäße medizinische Blutbehandlungseinrichtung zeichnet sich dadurch aus, dass neben der Vorrichtung zur extrakorporalen Blutbehandlung eine Vorrichtung zur elektrischen Muskelstimulation (EMS) vorgesehen ist, mit der sich während der Dialysebehandlung Muskeln des Patienten gezielt stimulieren lassen. Hierzu verfügt die Vorrichtung zur elektrischen Muskelstimulation (EMS) über eine Einrichtung zur Erzeugung von elektrischen Impulsen und eine oder mehrere Elektroden, die an der Einrichtung zur Erzeugung von elektrischen Impulsen angeschlossen sind. Um Muskeln des Patienten, insbesondere Muskeln im Beinbereich, gezielt stimulieren zu können, werden die Elektroden auf die Haut des Patienten, insbesondere die Beine, aufgelegt.

Es hat sich gezeigt, dass eine Muskelstimulation während der Blutbehandlung, beispielsweise Hämodialysebehandlung, zu einer verbesserten Verträglichkeit der Dialysebehandlung für den Patienten und einer Steigerung der Effizienz der Behandlung führt. Dies ist mit einem verbesserten Übergang von "Schlackestoffen" aus dem Muskelgewebe in die Blutbahn zu erklären.

Von Vorteil ist, dass die erfindungsgemäße Blutbehandlungseinrichtung eine Verbesserung der Effizienz der Blutbehandlung, beispielsweise der Hämodialysebehandlung, ohne aktive sportliche Betätigung des Patienten erlaubt. Dadurch kann die Effizienz der Dialysebehandlung auch von Patienten, die nicht über eine ausreichende körperliche Leistungsfähigkeit verfügen, verbessert werden. Da die Verbesserung der Dialyseeffizienz nicht die aktive Betätigung des Patienten voraussetzt, wird darüber hinaus nicht das Risiko einer Nadeldiskonnektion erhöht. Weiterhin ist zusätzliches Personal für behandlungsbegleitende Massagen nicht erforderlich.

Die Vorrichtung zur extrakorporalen Blutbehandlung und die Vorrichtung zur elektrischen Muskelstimulation werden nicht unabhängig voneinander betrieben, sondern beide Vorrichtungen kommunizieren während der Blutbehandlung miteinander.

Die Vorrichtung zur extrakorporalen Blutbehandlung verfügt über eine Eingabeeinheit zur Eingabe von für die Blutbehandlung relevanten Parametern, zu denen sowohl die für den Patienten spezifischen Parameter, beispielsweise das Alter und Geschlecht des Patienten, der Blutdruck, Hämatokrit oder dgl., als auch die für die Blutbehandlung relevanten Betriebsparameter der extrakorporalen Blutbehandlungsvorrichtung zählen, beispielsweise Blutfluss- und Dialysierflüssigkeitsrate oder Ultrafiltrationsrate etc.

Weiterhin verfügt die extrakorporale Blutbehandlungsvorrichtung über eine Steuereinheit für die Steuerung von Komponenten zur Durchführung der Blutbehandlung und/oder Überwachung der für die Blutbehandlung relevanten Parameter. Zu diesen Komponenten gehören beispielsweise die Blutpumpe zum Fördern des Bluts im extrakorporalen Blutkreislauf mit der vorgegebenen Blutflussrate oder die Dialysierflüssigkeitspumpe zum Fördern der Dialysierflüssigkeit mit der vorgegebenen Dialysierflüssigkeitsrate. Zu den während der Blutbehandlung zu überwachenden Parametern zählen beispielsweise die Blutfluss- und Dialysierflüssigkeitsrate oder Ultrafiltrationsrate.

Die Vorrichtung zur elektrischen Muskelstimulation verfügt über eine Steuereinheit zur Steuerung der Einrichtung zur Erzeugung von elektrischen Impulsen. Diese Steuereinheit ist vorzugsweise derart ausgebildet, dass die Impulsform und/oder Stromstärke und/oder Amplitude und/oder Frequenz und/oder Breite und/oder die Modulationszeit der Impulse einstellbar ist.

Die Steuereinheit für die Steuerung der Komponenten zur Durchführung und/oder Überwachung der Blutbehandlung wirkt mit der Steuereinheit zur Steuerung der Einrichtung zur Erzeugung von elektrischen Impulsen derart zusammen, dass die Erzeugung der elektrischen Impulse in Abhängigkeit von den für die Blutbehandlung relevanten Parametern steuerbar ist. Dadurch ist es möglich, in Abhängigkeit von den für die Blutbehandlung relevanten Parametern die Vorrichtung zur elektrischen Muskelstimulation im Sinne eines optimalen Behandlungsergebnisses zu steuern und/oder zu regeln. Es ist aber auch möglich, in Abhängigkeit von den Vorgaben für die Erzeugung der elektrischen Impulse, beispielsweise der Stromstärke, Amplitude, Frequenz, Breite der Impulse etc., die von der Steuereinheit der Einrichtung zur Erzeugung von elektrischen Impulsen vorgegeben werden, die extrakorporale Blutbehandlungsvorrichtung zu steuern und/oder zu regeln.

Eine Ausführungsform der medizinischen Blutbehandlungseinrichtung sieht vor, dass die Vorrichtung zur extrakorporalen Blutbehandlung und die Vorrichtung zur elektrischen Muskelstimulation als eine Geräteeinheit ausgebildet sind. Beispielsweise können beide Vorrichtungen über eine gemeinsame Steuereinheit (Mikroprozessor) verfügen. Es ist aber auch möglich, dass die Vorrichtung zur extrakorporalen Blutbehandlung und die Vorrichtung zur elektrischen Muskelstimulation als zwei separate Geräteeinheiten ausgebildet sind, die miteinander kommunizieren können. Hierzu verfügen die Vorrichtung zur extrakorporalen Blutbehandlung und die Vorrichtung zur elektrischen Muskelstimulation vorzugsweise jeweils über eine Einheit zum Austausch von für die Blutbehandlung relevanten Parametern, die zur Übertragung dieser Parameter miteinander verbunden sind. Die Einheiten zum Austausch der für die Blutbehandlung, relevanten Parameter können als Einheiten zur unidirektionalen Übertragung von Daten von der extrakorporalen Blutbehandlungsvorrichtung auf die Vorrichtung zur elektrischen Muskelstimulation oder von der Vorrichtung zur elektrischen Muskelstimulation auf die extrakorporale Blutbehandlungsvorrichtung ausgebildet sein. Es ist auch eine bidirektionale Datenübertragung zwischen beiden Vorrichtungen möglich, die sowohl eine Steuerung und/oder Regelung der Vorrichtung zur elektrischen Muskelstimulation in Abhängigkeit von dem Betriebszustand der extrakorporalen Blutbehandlungsvorrichtung und/oder die Steuerung und/oder Regelung der extrakorporalen Blutbehandlungsvorrichtung in Abhängigkeit von dem Betriebszustand der Vorrichtung zur elektrischen Muskelstimulation erlaubt.

Bei einer bevorzugten Ausführungsform erfolgt der Austausch der für die Blutbehandlung relevanten Parameter zwischen beiden Vorrichtungen über eine drahtgebundene Verbindung. Hierzu werden die beiden Geräte, die über konventionelle Schnittstellen verfügen können, mittels eines konventionellen Verbindungskabels miteinander verbunden.

Eine alternative Ausführungsform sieht eine drahtlose Verbindung, insbesondere eine Funkverbindung zwischen beiden Vorrichtungen vor. Eine drahtlose Datenübertragung ist auch mit einer optischen Verbindung möglich.

Eine weitere besonders bevorzugte Ausführungsform sieht vor, dass die Elektroden und/oder die Anschlüsse für die Elektroden der Vorrichtung zur elektrischen Muskelstimulation in eine Einrichtung zum Liegen und/oder Sitzen des Patienten integriert sind. Dadurch liegen die Elektroden immer für den Anschluss an den Patienten bereit, und es ist besonders einfach möglich, die Elektroden an den Patienten anzuschließen.

**Die** erfindungsgemäße **Vorrichtung** zur extrakorporalen Blutbehandlung zeichnet sich dadurch aus, dass während der extrakorporalen Blutbehandlung, bei der Blut eines Patienten in einem extrakorporalen Blutkreislauf zu einer Blutbehandlungseinheit strömt und Blut aus der Blutbehandlungseinheit zurück zum Patienten strömt, Muskeln des Patienten mittels elektrischer Impulse behandlungsbegleitend gezielt stimuliert werden.

Im Folgenden werden Ausführungsbeispiele der Erfindung unter Bezugnahme auf die Zeichnungen näher erläutert.

Es zeigen:
- Fig. 1: ein Ausführungsbeispiel der erfindungsgemäßen medizinischen Blutbehandlungseinrichtung mit einer Vorrichtung zur extrakorporalen Blutbehandlung und einer Vorrichtung zur elektrischen Muskelstimulation in stark vereinfachter schematischer Darstellung und
- Fig. 2: ein zweites Ausführungsbeispiel der erfindungsgemäßen medizinischen Blutbehandlungseinrichtung in stark vereinfachter schematischer Darstellung.

Die medizinische Blutbehandlungseinrichtung verfügt über eine Vorrichtung zur extrakorporalen Blutbehandlung A und eine Vorrichtung zur elektrischen Muskelstimulation (EMS) B.

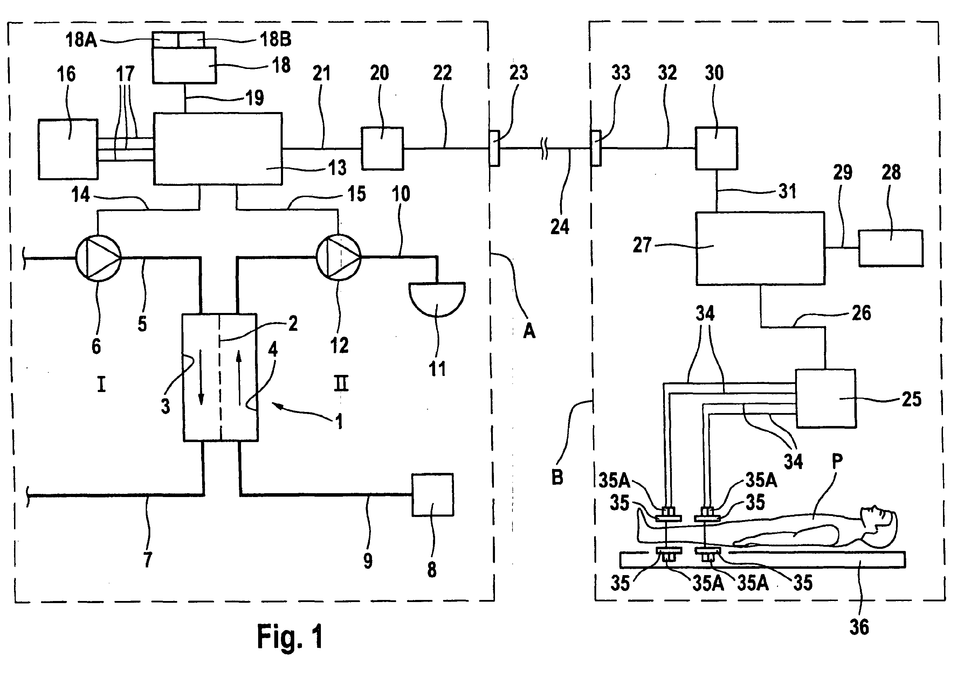

Bei dem Ausführungsbeispiel von Fig. 1 sind die extrakorporale Blutbehandlungsvorrichtung und die Vorrichtung zur elektrischen Muskelstimulation als zwei separate Geräteeinheiten A, B ausgebildet, die miteinander kommunizieren können. Bei der extrakorporalen Blutbehandlungsvorrichtung A handelt es sich bei dem vorliegenden Ausführungsbeispiel um eine Hämodialysevorrichtung. Die Dialysevorrichtung weist einen Dialysator 1 auf, der durch eine semipermeable Membran 2 in eine Blutkammer 3 und eine Dialysierflüssigkeitskammer 4 unterteilt ist. An dem Einlass der Blutkammer 3 ist eine arterielle Blutleitung 5 angeschlossen, in die eine Blutpumpe 6 geschaltet ist. Von dem Auslass der Blutkammer 3 geht eine venöse Blutleitung 7 ab. Die arterielle und venöse Kanüle zum Anschluss der Dialysevorrichtung an den Patienten sind der besseren Übersichtlichkeit halber nicht dargestellt.

In einer Dialysierflüssigkeitsquelle 8 wird frische Dialysierflüssigkeit bereitgestellt. Von der Dialysierflüssigkeitsquelle 8 führt eine Dialysierflüssigkeitszuführleitung 9 zu dem Einlass der Dialysierflüssigkeitskammer 4 des Dialysators 1, während eine Dialysierflüssigkeitsabführleitung 10 von dem Auslass der Dialysierflüssigkeitskammer zu einem Abfluss 11 führt. In die Dialysierflüssigkeitsabführleitung 10 ist eine Dialysierflüssigkeitspumpe 12 geschaltet.

Während der Hämodialyse strömt Blut des Patienten im extrakorporalen Blutkreislauf I durch die Blutkammer 3 des Dialysators 1, während Dialysierflüssigkeit im Dialysierflüssigkeitssystem II durch die Dialysierflüssigkeitskammer 4 des Dialysators 1 strömt.

Die Dialysevorrichtung A verfügt über eine Steuereinheit 13, die mit der Blutpumpe 6 und der Dialysierflüssigkeitspumpe 12 über Steuerleitungen 14, 15 verbunden ist. Die Steuereinheit 13 gibt die Drehzahl der Blut- und Dialysierflüssigkeitspumpe 6, 12 vor, so dass sich ein bestimmter Blut- und Dialysierflüssigkeitsfluss einstellt. Neben der Blut- und Dialysierflüssigkeitspumpe verfügt die Dialysevorrichtung noch über weitere Komponenten zur Durchführung der Blutbehandlung, die aber der besseren. Übersicht halber wieder nicht dargestellt sind. Hierzu zählen beispielsweise eine Bilanziereinrichtung, Sperrorgane etc. Auch diese Komponenten werden von der Steuereinheit 13 angesteuert, um die Dialysebehandlung durchführen zu können. Anstelle der Steuereinheit 13 für die Dialysevorrichtung A kann auch eine Regeleinheit vorgesehen oder die Einrichtung als Einheit zum Steuern und/oder Regeln ausgebildet sein. Mit der Regeleinheit können auch Regelschleifen aufgebaut werden. Es können einige Parameter gesteuert, andere auch geregelt werden, so dass eine kombinierte Steuerung und/oder Regelung möglich ist.

Die Steuereinheit 13 steuert nicht nur die zur Durchführung der Blutbehandlung erforderlichen Komponenten an, sondern überwacht auch die für die Dialysebehandlung relevanten Parameter. Die für die Blutbehandlung relevanten Parameter werden von verschiedenen nur schematisch dargestellten Sensoren 16 erfasst, die über Datenleitungen 17 mit der Steuereinheit 13 verbunden sind

Darüber hinaus weist die Dialysevorrichtung eine Eingabeeinheit 18 auf, mit der die für die Blutbehandlung relevanten Parameter eingegeben werden können. Die Parameter können beispielsweise mit einer Tastatur 18A manuell eingegeben oder mit einer Speicherkarte 18B (Patientenkarte) eingelesen werden. Die Eingabeeinheit 18 ist mit einer Datenleitung 19 wiederum mit der Steuereinheit 13 verbunden.

Zur Kommunikation mit der Vorrichtung zur elektrischen Muskelstimulation (EMS) B weist die Dialysevorrichtung A eine Einheit 20 vorzugsweise zur bidirektionalen Übertragung von für die Blutbehandlung relevanten Parametern auf, die über eine Datenleitung 21 mit der Steuereinheit 13 verbunden ist. Bei der Einheit 20 kann es sich um eine konventionelle Schnittstelle handeln, die über eine Datenleitung 22 mit einem Stecker 23 verbunden ist, an den ein Datenkabel 24 angeschlossen werden kann. Es ist aber auch möglich, dass die Einheit 20 einen Sender/Empfänger zur Übertragung der Daten als Funksignale oder optische Signale aufweist. In diesem Fall entfällt die Leitung 22 und der Stecker 23.

Die Vorrichtung B zur elektrischen Muskelstimulation verfügt über eine Einrichtung 25 zur Erzeugung von elektrischen Impulsen, die der Stimulation der Muskeln des Patienten während der Blutbehandlung dienen. Die Einrichtung 25 zur Erzeugung der elektrischen Impulse ist über eine Steuerleitung 26 mit einer Steuereinheit 27 verbunden. Die Steuereinheit 27 steuert die Einrichtung 25 derart an, dass die elektrischen Impulse eine bestimmte Impulsform, Stromstärke, Amplitude, Frequenz und Breite haben. Auch ist die Modulationszeit der Impulse einstellbar. Die Impulsform charakterisiert den zeitlichen Verlauf eines Einzelimpulses. Beispielsweise werden Rechteckimpulse erzeugt. Die Stromstärke sollte stets so eingestellt sein, dass eine ausreichende Muskelkontraktion erfolgt, auf jeden Fall sollte die Muskelstimulation aber (weit) unterhalb der Schmerzensgrenze liegen. Unter der Amplitude wird die maximale Stromstärke eines Einzelimpulses verstanden. Die Impulsbreite gibt die zeitliche Dauer eines Einzelimpulses an und bestimmt neben der Amplitude die Stärke der Muskelkontraktion. Unter der Frequenz versteht man die Anzahl der Einzelimpulse pro Sekunde. Vorzugsweise werden Frequenzen in einem Bereich von ca. 2 bis 90 Herz eingestellt. Darüber hinaus können auch die Modulationszeiten eingestellt werden, die den zeitlichen Ablauf der Amplituden der Einzelimpulse angeben.

Die oben genannten Größen können mit einer Eingabeeinheit 28, beispielsweise einer Tastatur manuell eingegeben werden, die über eine Datenleitung 29 mit der Steuereinheit 27 verbunden ist.

Anstelle der Steuereinheit 27 für die Vorrichtung B zur elektrischen Muskelstimulation kann auch eine Regeleinheit vorgesehen oder die Einrichtung als Einheit zum Steuern und/oder Regeln ausgebildet sein. Mit der Regeleinheit können auch Regelschleifen aufgebaut werden. Es können einige Parameter gesteuert, andere auch geregelt werden, so dass eine kombinierte Steuerung und/oder Regelung möglich ist.

Die Vorrichtung B zur elektrischen Muskelstimulation verfügt über eine Einheit 30 zur Übertragung der für die Behandlung vorgesehenen Daten zu der Dialysevorrichtung A oder zum Empfangen von Daten von der Dialysevorrichtung. Bei der Einheit 30, die über eine Datenleitung 31 mit der Steuereinheit 27 verbunden ist, handelt es sich beim vorliegenden Ausführungsbeispiel wieder um eine konventionelle Schnittstelle zur bidirektionalen Datenübertragung, an der über eine Datenleitung 32 ein Stecker 33 angeschlossen ist. An den Stecker 33 kann das Datenkabel 24 angeschlossen werden, so dass die Dialysevorrichtung A und die Vorrichtung B zur elektrischen Muskelstimulation miteinander kommunizieren können. Anstelle einer drahtgebundenen Drahtübertragung ist auch eine drahtlose Datenübertragung möglich. Für diesen Fall weist die Einheit 30 einen Sender/Empfänger auf, die Funksignale oder optische Signale senden und empfangen können. Damit entfällt die Datenleitung 32 und der Stecker 33.

An der Einrichtung 25 zur Erzeugung von elektrischen Impulsen sind über Leitungen 34 eine Mehrzahl von Elektroden 35 angeschlossen, die auf die Haut des Patienten P, insbesondere auf die Beine des Patienten aufgelegt werden.

Während der Blutbehandlung sitzt und/oder liegt der Patient P. Die Einrichtung zum Sitzen und/oder Liegen während der Behandlung, d.h. der Sessel oder das Bett 36, auf dem der Patient sitzt und/oder liegt, ist nur schematisch dargestellt. An dem Sessel oder Bett 36 sind Anschlüsse 35A vorgesehen, an denen die Elektroden 35, die auf die Haut des Patienten aufgelegt werden, gegebenenfalls über kurze in Fig. 1 nicht dargestellte Verbindungskabel angeschlossen werden. Die Anschlüsse 35A für die Elektroden 35, beispielsweise Stecker oder Buchsen, sind mit den Leitungen 34 verbunden. Vorzugsweise sind die Anschlüsse 35A für die Elektroden 35 an der oder in die Sitz- oder Liegefläche fest integriert. Grundsätzlich ist es aber auch möglich, die Elektroden selbst in die Sitz- oder Liegefläche zu integrieren. Dann muss aber für einen sicheren Kontakt der Elektroden mit der Haut des Patienten gesorgt werden.

Die Steuereinheit 13 der Blutbehandlungsvorrichtung A und die Steuereinheit 27 der Vorrichtung B zur elektrischen Muskelstimulation sind derart ausgebildet, dass in Abhängigkeit von den für die Blutbehandlung relevanten Parametern, die mit der Eingabeeinheit 18 eingegeben werden können und/oder während der Blutbehandlung von den Sensoren 16 erfasst werden, die Impulsform und/oder Stromstärke und/oder Amplitude und/oder Frequenz und/oder Breite und/oder Modulationszeit der Impulse eingestellt wird. Beispielsweise können zu Beginn der Behandlung elektrische Impulse mit einer anderen Impulsform, Stromstärke, Amplitude, Frequenz, Breite oder Modulationszeit erzeugt werden als zum Ende der Behandlung. Es ist auch möglich, dass in Abhängigkeit von den für den Patienten spezifischen Parametern, die mit der Patientenkarte eingelesen oder auch manuell eingegeben werden_können,_elektrische Impulse mit einer bestimmten Form, Stärke, Amplitude etc. erzeugt werden. Die elektrische Muskelstimulation kann beispielsweise so betrieben werden, dass die Behandlungszeit minimiert wird. Da während der Blutbehandlung die einzelnen Parameter überwacht werden, können auch die Auswirkungen der elektrischen Muskelstimulation auf die Blutbehandlung festgestellt werden. Eine bevorzugte Ausführungsform sieht beispielsweise vor, dass die Steuereinheit 13 der Dialysevorrichtung A in Abhängigkeit von der Art der elektrischen Impulse (Impulsform, Stromstärke, Amplitude etc.) und den Auswirkungen der elektrischen Muskelstimulation die für die Blutbehandlung relevanten Parameter, wie Behandlunsgzeit, Dialysierflüssigkeitsrate etc. im Sinne einer optimalen Behandlung einstellt. Dabei kann die Steuereinheit 13 auch lernfähig sein.

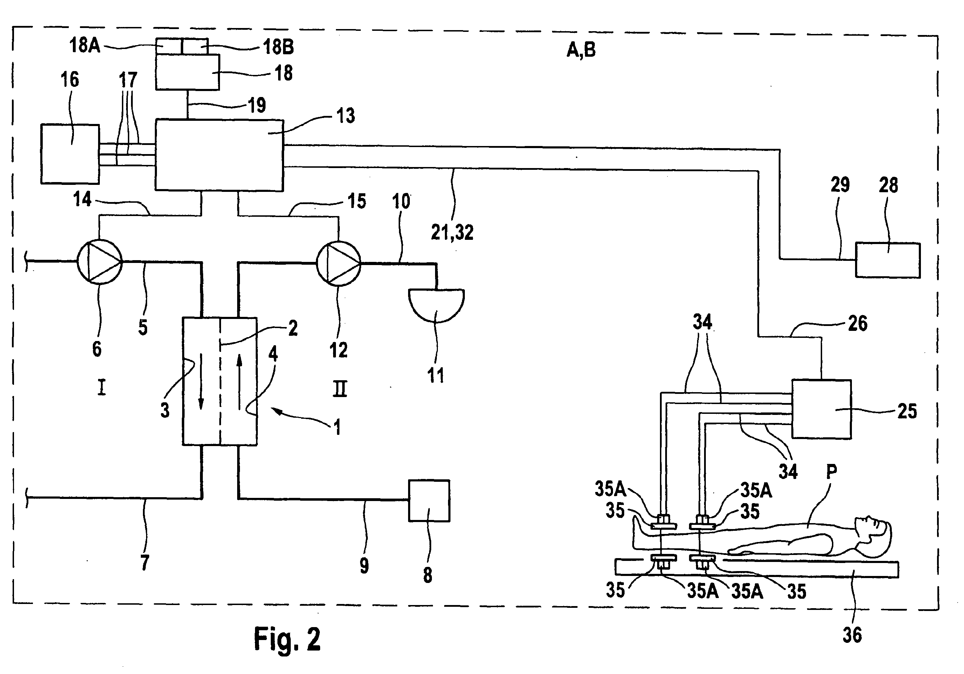

Fig. 2 zeigt ein alternatives Ausführungsbeispiel der medizinischen Blutbehandlungseinrichtung, die sich von der Behandlungseinrichtung von Fig. 1 dadurch unterscheidet, dass die extrakorporale Dialysevorrichtung A und die Vorrichtung B zur elektrischen Muskelstimulation nicht als zwei separate Geräteeinheiten ausgebildet sind, sondern eine Geräteeinheit bilden. Die einander entsprechenden Teile sind mit den gleichen Bezugsziffern bezeichnet.

Bei der alternativen Ausführungsform machen die extrakorporale Blutbehandlungsvorrichtung A und die Vorrichtung B zur elektrischen Muskelstimulation von derselben Steuer- und/oder Regeleinheit 13 Gebrauch, an der auch die Eingabeeinheit 28 der Vorrichtung B zur elektrischen Muskelstimulation über die Leitung 29 angeschlossen ist. Diese Steuer- und Regeleinheit 13 ist vorzugsweise die bereits in den bekannten Blutbehandlungsvorrichtungen vorgesehene Steuereinheit (Mikroprozessor). Da getrennte Steuer- und Regeleinheiten nicht vorgesehen sind, können bei diesem Ausführungsbeispiel auch die Schnittstellen entfallen. Es ist auch möglich, dass die Eingabeeinheiten 18 und 28 zu einer gemeinsamen Eingabeeinheit zusammengefasst werden.

## Patentansprüche

1. Medizinische Blutbehandlungseinrichtung mit
einer Vorrichtung (A) zur extrakorporalen Blutbehandlung, und
einer Vorrichtung (B) zur elektrischen Muskelstrmulation (EMS), die eine Einrichtung (25) zur Erzeugung von elektrischen Impulsen, die der elektrischen Stimulation der Muskeln dienen, und eine oder mehrere Elektroden (35) aufweist, die an der Einrichtung zur Erzeugung von elektrischen Impulsen angeschlossen sind, wobei
die Vorrichtung (A) zur extrakorporalen Blutbehandlung eine Eingabeeinheit (18) zur Eingabe von für die Blutbehandlung relevanten Parametern und eine Steuer- und/oder Regeleinheit (13) für die Steuerung und/oder Regelung von Komponenten zur Durchführung der Blutbehandlung und/oder Überwachung von für die Blutbehandlung relevanten Parametern aufweist,
**dadurch gekennzeichnet, dass** die Vorrichtung (B) zur elektrischen Muskelstimulation eine Steuer- und/oder Regeleinheit (27) zur Steuerung und/oder Regelung der Einrichtung (25) zur Erzeugung von elektrischen Impulsen aufweist, wobei
die Steuer- und/oder Regeleinheit (13) für die Steuerung und/oder Regelung der Komponenten zur Durchführung und/oder Überwachung der Blutbehandlung mit der Steuer- und/oder Regeleinheit (27) zur Steuerung und/oder Regelung der Einrichtung (25) zur Erzeugung von elektrischen Impulsen derart zusammenwirkt, dass die Erzeugung der elektrischen Impulse in Abhängigkeit von den für die Blutbehandlung relevanten Parametern steuer- und/oder regelbar ist.

2. Medizinische Blutbehandlungseinrichtung nach Anspruch 1, **dadurch gekennzeichnet, dass** die Steuer- und/oder Regeleinheit (27) zur Steuerung und/oder Regelung der Einrichtung (25) zur Erzeugung von elektrischen Impulsen derart ausgebildet ist, dass die Impulsform und/oder Stromstärke und/oder Amplitude und/oder Frequenz und/oder Breite und/oder die Modulationszeit der Impulse einstellbar ist.

3. Medizinische Blutbehandlungseinrichtung nach Anspruch 1 oder 2, **dadurch gekennzeichnet, dass** die Vorrichtung (A) zur extrakorporalen Blutbehandlung und die Vorrichtung (B) zur elektrischen Muskelstimulation als eine Geräteeinheit ausgebildet sind.

4. Medizinische Blutbehandlungseinrichtung nach Anspruch 1 oder 2, **dadurch gekennzeichnet, dass** die Vorrichtung (A) zur extrakorporalen Blutbehandlung und die Vorrichtung (B) zur elektrischen Muskelstimulation als zwei separate Geräteeinheiten ausgebildet sind, wobei die Vorrichtung zur extrakorporalen Blutbehandlung eine Einheit (20) zum Austausch von für die Blutbehandlung relevanten Parametern und die Vorrichtung zur elektrischen Muskelstimulation eine Einheit (30) zum Austausch von für die Behandlung relevanten Parametern aufweisen, die zur Übertragung der für die Behandlung relevanten Parameter miteinander verbindbar sind.

5. Medizinische Blutbehandlungseinrichtung nach Anspruch 4, **dadurch gekennzeichnet, dass** die Einheiten (20, 30) zum Austausch von für die Blutbehandlung relevanten Parametern der Vorrichtung (A) zur extrakorporalen Blutbehandlung und der Vorrichtung (B) zur elektrischen Muskelstimulation als Einheiten zur unidirektionalen Übertragung von für die Behandlung vorgesehenen Daten von der Vorrichtung zur extrakorporalen Blutbehandlung auf die Vorrichtung zur elektrischen Muskelstimulation ausgebildet sind.

6. Medizinische Blutbehandlungseinrichtung nach Anspruch 4, **dadurch gekennzeichnet, dass** die Einheiten (20, 30) zum Austausch von für die Blutbehandlung relevanten Parametern der Vorrichtung (A) zur extrakorporalen Blutbehandlung und der Vorrichtung (B) zur elektrischen Muskelstimulation als Einheiten zur unidirektionalen Übertragung von für die Behandlung vorgesehenen Daten von der Vorrichtung zur elektrischen Muskelstimulation auf die Vorrichtung zur extrakorporalen Blutbehandlung ausgebildet sind.

7. Medizinische Blutbehandlungseinrichtung nach Anspruch 4, **dadurch gekennzeichnet, dass** die Einheiten (20, 30) zum Austausch von für die Blutbehandlung relevanten Parametern der Vorrichtung (A) zur extrakorporalen Blutbehandlung und der Vorrichtung (B) zur elektrischen Muskelstimulation als Einheiten zur bidirektionalen Übertragung von für die Behandlung vorgesehenen Daten ausgebildet sind.

8. Medizinische Blutbehandlungseinrichtung nach einem der Ansprüche 4 bis 7, **dadurch gekennzeichnet, dass** die Verbindung zwischen den Einheiten (20, 30) zum Austausch von für die Blutbehandlung relevanten Parametern der Vorrichtung zur extrakorporalen Blutbehandlung und der Vorrichtung zur elektrischen Muskelstimulation als eine drahtgebundene Verbindung ausgebildet ist.

9. Medizinische Blutbehandlungseinrichtung nach einem der Ansprüche 4 bis 7, **dadurch gekennzeichnet, dass** die Verbindung zwischen den Einheiten (20, 30) zum Austausch von für die Behandlung relevanter Parameter der Vorrichtung zur extrakorporalen Blutbehandlung und der Vorrichtung zur elektrischen Muskelstimulation als eine drahtlose Verbindung, insbesondere eine Funkverbindung, ausgebildet ist.

10. Medizinische Blutbehandlungseinrichtung nach einem der Ansprüche 1 bis 9, **dadurch gekennzeichnet, dass** die Anschlüsse (35A) für die eine oder mehreren Elektroden (35) in eine Einrichtung (36) zum Liegen und/oder Sitzen des Patienten integriert sind.

11. Vorrichtung zur extrakorporalen Blutbehandlung mit einer Eingabeeinheit (18) zur Eingabe von für die Blutbehandlung relevanten Parametern und einer Steuer- und/oder Regeleinheit (13) für die Steuerung und/oder Regelung von Komponenten zur Durchführung der Blutbehandlung, und/oder Überwachung von für die Blutbehandlung relevanten Parametern, **dadurch gekennzeichnet, dass** die extrakorporale Blutbehandlungsvorrichtung eine Einheit (20) zum Austausch von für die Blutbehandlung relevanten Parametern mit einer Vorrichtung zur elektrischen Muskelsümulation aufweist, die eine Einrichtung zur Erzeugung von elektrischen Impulse, die der elektrischen Stimulation der Muskeln dienen, und eine oder mehrere Elektroden aufweist, die an der Einrichtung zur Erzeugung von elektrischen Impulsen angeschlossen sind,
wobei die Steuer- und/oder Regeleinheit (13) zur Steuerung und/oder Regelung von Komponenten zur Durchführung und/oder Überwachung der Blutbehandlung derart ausgebildet ist, dass die Erzeugung der elektrischen Impulse zur elektrischen Stimulation der Muskeln in Abhängigkeit von den für die Blutbehandlung relevanten Parametern steuer- und/oder regelbar ist.

12. Vorrichtung zur elektrischen Muskelstimulation, die eine Einrichtung (25) zur Erzeugung von elektrischen Impulsen, die der elektrischen Stimulation der Muskeln dienen, und eine oder mehrere Elektroden (35) aufweist, die an der Einrichtung (25) zur Erzeugung von elektrischen Impulsen angeschlossen sind,
**dadurch gekennzeichnet, dass** die Vorrichtung zur elektrischen Muskelstimulation eine Einheit (30) zum Austausch von für die Blutbehandlung relevanten Parametern mit einer Vorrichtung zur extrakorporalen Blutbehandlung aufweist. die eine Eingabeeinheit zur Eingabe von für die Blutbehandlung relevanten Parametern und eine Steuer- und/oder Regeleinheit zur Steuerung und/oder Regelung von Komponenten zur Durchführung der Blutbehandlung und/oder Überwachung von für die Blutbehandlung relevanten Parametern aufweist,
wobei die Vorrichtung zur elektrischen Muskelstimulation eine Steuer- und/oder Regeleinheit (27) zur Steuerung und/oder Regelung der Einrichtung (25) zur Erzeugung von elektrischen Impulsen aufweist, die derart ausgebildet ist, dass die Erzeugung der elektrischen Impulse zur elektrischen Stimulation der Muskeln in Abhängigkeit von den für die Blutbehandlung relevanten Parametern steuer- und/oder regelbar ist.

13. Vorrichtung nach Anspruch 11 oder 12, **dadurch gekennzeichnet, dass** die Einheit (20 bzw. 30) zum Austausch von für die Blutbehandlung relevanten Parametern der Vorrichtung (A) zur extrakorporalen Blutbehandlung, bzw. der Vorrichtung (B) zur Muskelstimulation als Einheit zur unidirektionalen oder bidirektionalen Übertragung von für die Behandlung vorgesehenen Daten zwischen der Vorrichtung zur elektrischen Muskelstimulation und der Vorrichtung zur extrakorporalen Blutbehandlung ausgebildet sind.

14. Vorrichtung nach einem der Ansprüche 11 bis 13, **dadurch gekennzeichnet, dass** die Verbindung zwischen den Einheiten (20 bzw. 30) zum Austausch von für die Blutbehandlung relevanten Parametern der Vorrichtung (A) zur extrakorporalen Blutbehandlung bzw. der Vorrichtung (B) zur elektrischen Muskelstimulation als eine drahtgebundene oder drahtlose Verbindung, insbesondere eine Funkverbindung ausgebildet ist.

## Claims

1. A medical blood treatment apparatus comprising
a device (A) for extracorporeal blood treatment, und
a device (B) for electrical muscle stimulation (EMS), comprising means (25) for generating electrical pulses used for the electrical stimulation of the muscles and one or more electrodes (35) which are connected to the means for generating electrical pulses, wherein
the device (A) for extracorporeal blood treatment comprises an input unit (18) for inputting parameters relevant for the blood treatment und an open- and/or closed-loop control unit (13) for the open- and/or closed-loop control of components for carrying out the blood treatment and/or for monitoring parameters relevant for the blood treatment,
**characterised in that** the device (B) for electrical muscle stimulation comprises an open- and/or closed-loop control unit (27) for the open- and/or closed-loop control of the means (25) for generating electrical pulses, wherein
for carrying out and/or monitoring the blood treatment, the open- and/or closed-loop control unit (13) for the open- and/or closed-loop control of the components cooperates with the open- and/or closed-loop control unit (27) for the open- and/or closed-loop control of the means (25) for generating electrical pulses in such a way that the generation of the electrical pulses can be open- and/or closed-loop controlled as a function of the parameters relevant for the blood treatment.

2. The medical blood treatment apparatus as claimed in claim 1, **characterised in that** the open- and/or closed-loop control unit (27) is designed for the open- and/or closed-loop control of the means (25) for generating electrical pulses in such a way that the pulse shape and/or the current strength and/or the amplitude and/or the frequency and/or the width and/or the modulation time of the pulses can be adjusted.

3. The medical blood treatment apparatus as claimed in claim 1 or 2, **characterised in that** the device (A) for extracorporeal blood treatment and the device (B) for electrical muscle stimulation are designed as a single unit.

4. The medical blood treatment apparatus as claimed in claim 1 or 2, **characterised in that** the device (A) for extracorporeal blood treatment and the device (B) for electrical muscle stimulation are designed as two separate units, wherein the device for extracorporeal blood treatment comprises a unit (20) for exchanging parameters relevant for the blood treatment and the device for electrical muscle stimulation comprises a unit (30) for exchanging parameters relevant for the treatment, which units can be connected to each other for transferring the parameters relevant for the treatment.

5. The medical blood treatment apparatus as claimed in claim 4, **characterised in that** the units (20, 30) for exchanging parameters relevant for the blood treatment of the device (A) for extracorporeal blood treatment and the device (B) for electrical muscle stimulation are designed as units for the unidirectional transfer of data provided for the treatment from the device for extracorporeal blood treatment to the device for electrical muscle stimulation.

6. The medical blood treatment apparatus as claimed in claim 4, **characterised in that** the units (20, 30) for exchanging parameters relevant for the blood treatment of the device (A) for extracorporeal blood treatment and the device (B) for electrical muscle stimulation are designed as units for the unidirectional transfer of data provided for the treatment from the device for electrical muscle stimulation to the device for extracorporeal blood treatment.

7. The medical blood treatment apparatus as claimed in claim 4, **characterised in that** the units (20, 30) for exchanging parameters relevant for the blood treatment of the device (A) for extracorporeal blood treatment and the device (B) for electrical muscle stimulation are designed as units for the bidirectional transfer of data provided for the treatment.

8. The medical blood treatment apparatus as claimed in any one of claims 4 to 7, **characterised in that** the connection between the units (20, 30) for exchanging parameters relevant for the blood treatment of the device for extracorporeal blood treatment and the device for electrical muscle stimulation is designed as a wired connection.

9. The medical blood treatment apparatus as claimed in any one of claims 4 to 7, **characterised in that** the connection between the units (20, 30) for exchanging parameters relevant for the blood treatment of the device for extracorporeal blood treatment and the device for electrical muscle stimulation is designed as a wireless connection, in particular a radio link.

10. The medical blood treatment apparatus as claimed in any one of claims 1 to 9, **characterised in that** the terminals (35A) for the one or more electrodes (35) are integrated into an installation (36) for the patient to lie and/or sit on.

11. A device for extracorporeal blood treatment, comprising an input unit (18) for inputting parameters relevant for blood treatment and an open- and/or closed-loop control unit (13) for the open- and/or closed-loop control of components for carrying out the blood treatment and/or for monitoring parameters relevant for the blood treatment, **characterised in that** the extracorporeal blood treatment device comprises a unit (20) for exchanging parameters relevant for the blood treatment with a device for electrical muscle stimulation, which comprises means for generating electrical pulses used for the electrical stimulation of the muscles and one or more electrodes connected to the means for generating electrical pulses,
wherein the open- and/or closed-loop control unit (13) for the open- and/or closed-loop control of components for carrying out and/or monitoring the blood treatment is designed in such a way that the generation of the electrical pulses for the electrical stimulation of muscles can be open- and/or closed-loop controlled as a function of the parameters relevant for the blood treatment.

12. A device for electrical muscle stimulation, comprising means (25) for generating electrical pulses used for the electrical stimulation of muscles and one or more electrodes (35) connected to the means (25) for generating electrical pulses,
**characterised in that** the device for electrical muscle stimulation comprises a unit (30) for exchanging parameters relevant for the blood treatment with a device for extracorporeal blood treatment, which comprises an input unit for inputting parameters relevant for the blood treatment and an open- and/or closed-loop control unit for the open- and/or closed-loop control of components for carrying out the blood treatment and/or for monitoring parameters relevant for the blood treatment,
wherein the device for electrical muscle stimulation comprises an open- and/or closed-loop control unit (27) for the open- and/or closed-loop control of the means (25) for generating electrical pulses, which is designed such that the generation of the electrical pulses for electrical muscle stimulation can be open- and/or closed-loop controlled as a function of the parameters relevant for the blood treatment.

13. The device as claimed in claim 11 or 12, **characterised in that** the units (20 and 30) for exchanging parameters relevant for the blood treatment of the device (A) for extracorporeal blood treatment and the device (B) for muscle stimulation are designed as a unit for the unidirectional or bidirectional transfer of data provided for the treatment between the device for electrical muscle stimulation and the device for extracorporeal blood treatment.

14. The device as claimed in any one of claims 11 to 13, **characterised in that** the connection between the units (20 and 30) for exchanging parameters relevant for the blood treatment of the device (A) for extracorporeal blood treatment and/or the device (B) for electrical muscle stimulation is designed as a wired or a wireless connection, in particular as a radio link.

## Revendications

1. Fraiseuse de route avec
un bâti de machine (1) et un mécanisme de roulement (2) qui présente des chenilles (2A et 2B) disposées sur les côtés avant et arrière du bâti de machine (1),
un dispositif de fraisage (5) disposé sous le bâti de machine (1) et
un poste de conduite (7) qui est disposé entre les chenilles (2A et 2B) disposées sur les côtés avant et arrière,
la fraiseuse de route présentant au moins une échelle (11) disposée sous le poste de conduite (7) sur le bâti de machine (1),
**caractérisée en ce que** l'échelle (11) est montée de manière à pouvoir pivoter sur un côté longitudinal (1C, 1D) du bâti de machine (1) depuis une position sensiblement verticale autour d'un axe de pivotement (27) s'étendant sensiblement transversalement au sens de la marche (10), l'échelle étant montée de manière pivotante sur un côté longitudinal (1C, 1D) du bâti de machine (1) de telle sorte que la partie de l'échelle se trouvant sous l'axe de pivotement (27) puisse dévier par un mouvement de pivotement dans le sens inverse au sens de la marche (10) lors de la butée contre un obstacle lorsque la fraiseuse de route se déplace dans le sens de la marche.

2. Fraiseuse de route selon la revendication 1, **caractérisée en ce que** l'axe de pivotement (27) s'étendant sensiblement transversalement au sens de la marche est en inclinaison par rapport à un côté longitudinal (1C, 1D) du bâti de machine depuis l'horizontale de sorte que l'échelle (11) soit inclinée dans la position verticale par rapport au côté longitudinal du bâti de machine (1).

3. Fraiseuse de route selon la revendication 1, **caractérisée en ce que** l'axe de pivotement (27) de l'échelle (11) est en inclinaison par rapport au plan défini par l'échelle de sorte que l'échelle (11) soit inclinée dans la position verticale par rapport au côté longitudinal du bâti de machine (1).

4. Fraiseuse de route selon l'une quelconque des revendications 1 à 3, **caractérisée en ce qu'**une liaison articulée (26) est prévue avec deux parties articulées (26A, 26B) rotatives l'une par rapport à l'autre autour d'un axe de rotation (27) s'étendant sensiblement transversalement au sens de la marche, le bâti de machine (1) présentant une partie articulée (26A) et l'échelle l'autre partie articulée (26B).

5. Fraiseuse de route selon la revendication 4, **caractérisée en ce que** la partie articulée (26B) de l'échelle (11) est disposée sur l'échelle (11) de telle manière que l'axe de rotation (27) de la liaison articulée (26) s'étende selon un angle aigu par rapport à un plan contenant l'échelle de sorte que l'échelle (11) se pose lors du pivotement depuis la position verticale sur un côté longitudinal (1C, 1D) du bâti de machine (1).

6. Fraiseuse de route selon l'une quelconque des revendications 1 à 5, **caractérisée en ce que** l'échelle (11) est montée de manière à pouvoir pivoter sur un côté longitudinal (1C, 1D) du bâti de machine (1) depuis la position verticale d'un angle sensiblement droit de sorte que l'échelle (11) puisse pivoter de la position verticale à une position horizontale.

7. Fraiseuse de route selon l'une quelconque des revendications 1 à 6, **caractérisée en ce que** l'échelle (11) est précontrainte de manière élastique dans la position horizontale et/ou verticale.

8. Fraiseuse de route selon l'une quelconque des revendications 1 à 7, **caractérisée en ce qu'**au moins un élément (11A) de l'échelle (11) présente un montant (12) avant dans le sens de la marche et un montant (13) arrière dans le sens de la marche, sur lesquels des barreaux (14, 15, 16, 17) sont fixés, le montant (12) avant dans le sens de la marche de l'échelle (11) étant fixé de manière pivotante sur un côté longitudinal du bâti de machine (1).

9. Fraiseuse de route selon la revendication 8, **caractérisée en ce que** le montant arrière (13) dans le sens de la marche de l'échelle (11) présente un élément de butée (28) qui s'appuie dans la position verticale de l'échelle sur un élément d'appui (24) qui est fixé sur un côté longitudinal (1C, 1D) du bâti de machine (1).

10. Fraiseuse de route selon la revendication 8, **caractérisée en ce que** le montant avant (12) dans le sens de la marche de l'échelle (11) présente un amortisseur de butée (32) qui s'appuie et se centre dans la position horizontale de l'échelle dans un perçage (33) qui est prévu dans un élément d'appui (24) sur un côté longitudinal (1C, 1D) du bâti de machine (1).

11. Fraiseuse de route selon l'une quelconque des revendications 1 à 10, **caractérisée en ce que** l'échelle (11) présente un élément supérieur et un élément inférieur (11A, 11B), l'élément inférieur (11B) étant fixé de manière pivotante sur l'élément supérieur (11A).

12. Fraiseuse de route selon la revendication 11, **caractérisée en ce que** l'élément supérieur (11A) de l'échelle (11) présente un montant (12) avant dans le sens de la marche et un montant (13) arrière dans le sens de la marche, sur lesquels des barreaux (14, 15, 16, 17) sont fixés, et l'élément inférieur (11B) de l'échelle (11) présente un barreau (19) fixé sur un montant (18), le montant (18) de l'élément inférieur (11B) pouvant pivoter d'une position dépliée à une position rabattue sur l'élément supérieur (11A), dans laquelle le barreau (19) de l'élément inférieur (11B) repose contre l'autre des deux montants (12) de l'élément supérieur (11A).

13. Fraiseuse de route selon la revendication 11 ou 12, **caractérisée en ce que** l'élément inférieur (11B) de l'échelle (11) présente un élément de fixation (23) avec lequel l'élément inférieur (11B) peut être arrêté dans la position repliée sur l'élément supérieur (11A).

14. Fraiseuse de route selon l'une quelconque des revendications 1 à 13, **caractérisée en ce qu'**un élément de retenue (30) qui dépasse dans la position verticale de l'échelle vers le haut, est fixé sur l'échelle (11).
